# EUROPEAN PATENT APPLICATION

(11) **EP 3 896 176 A1**
(43) Date of publication of application: **20.10.2021**
(21) Application number: 20185391.8
(22) Date of filing: 12.07.2020
(51) Int. Cl.: C12Q 1/6895

(54) **METHOD FOR THE DETERMINATION OF THE FINGERPRINT IN VARIETIES OF CANNABIS**

(30) Priority: 17.04.2020 WO PCT/ES2020/070248
(71) Applicant: Krei Method S.L., 43005 Tarragona (ES)
(72) Inventor: JIMÉNEZ BERNAL, Marco Antonio, 43005 Tarragona (ES)
(74) Representative: Cueto, Sénida

(57) **Abstract**

Method of obtaining a fingerprint from *Cannabis* samples comprising the following steps: determination of the genetic profile by analysis of at least one STR marker, obtaining the chemical profile by NMR, and differentiation of cannabis varieties from each other by comparing the results obtained in steps (a) and (b) on each sample with each other and/or with previously obtained fingerprint databases.

## Description

### FIELD OF THE INVENTION

The invention refers to the identification of plant samples. The present method comprises the molecular analysis of DNA markers and the determination of the chemical profile by means of ¹³C and ¹H NMR resonance patterns, of cannabis samples to achieve their forensic identification and traceability. Short tandem repeat analysis (STR) provides knowledge about the genetics of the sample, and nuclear magnetic resonance (NMR) analyses the phenotypic characteristics of the sample.

### BACKGROUND OF THE INVENTION

Despite the long historical relationship between cannabis and human society, there is no in-depth knowledge of the chemo-taxonomy and chemical composition of this plant. Seventy unique compounds, known collectively as phytocannabinoids, have been identified in cannabis plants¹.

There are estimated to be 104 naturally occurring cannabinoids in cannabis, the most studied ones being delta-9-tetrahydrocannabinol (Δ⁹-THC), delta-8-tetrahydrocannabinol (Δ⁸-THC), cannabidiol (CBD) and cannabinol (CBN)².
- THC produces psychoactive effects, such as euphoria, relaxation and the sharpening experiences sought by "recreational" cannabis users.
- CBD can moderate the psychoactive effects of THC and has antioxidant, antiinflammatory and neuroprotective effects.

Cannabis is commonly found in the following presentations:
- **Herbaceous:** consists of dry and crumbled leaves, in its flowering tops and in small stems.
- **Resin:** consists of the concentrated and pressed extract of the inflorescences and the plant.
- **Oil:** liquid concentrate obtained by mixing the resin of the plant with some organic solvent, resulting in a viscous mixture.
- **Waxes:** extracts from concentrated cannabis.
- **Edible preparations**: in the form of infusions, and foods such as chocolates, cookies, sweets, etc.

There is currently a debate about the number of species included in the genus *Cannabis.* The genus is generally considered by the scientific community to consist of the species *Cannabis sativa* L and its subspecies: *Cannabis sativa* L *sativa* subspecies and *Cannabis sativa* L *indica* subspecies. Four varieties: *Cannabis sativa* L. *sativa* subspecies *sativa* variety; *Cannabis sativa* L. *sativa* subspecies spontaneous variety *Vavilov; Cannabis sativa* L. *indica* subspecies *indica* (Lam.) Wehmer variety; *Cannabis sativa* L. *indica* subspecies *kafiristanica* variety (Vavilov). Although the drug is considered to be monospecific (*Cannabis sativa* L)³, psychoactive substances can be obtained from all the above-mentioned species, subspecies and varieties.

In 2009, the Latin American Commission on Drugs and Democracy published a report that questioned the effectiveness of "prohibitionist policies based on the repression of production and interdiction of trafficking and distribution, as well as the criminalization of consumption, have not produced the expected results. "We are further away than ever from the proclaimed objective of eradicating drugs"⁴.

The country that pioneered the full legalization of the production and sale of cannabis for recreational use was Uruguay, which was later joined by numerous countries such as Canada, Jamaica, Australia, Georgia, the Netherlands and South Africa, all of which have different nuances in their regulation.

The acceptance of "medical cannabis" is more widespread. Its use is regulated in more than 40 countries, among which the following ones stand out: Germany, Brazil, Colombia, Croatia, Denmark, Estonia and United Kingdom among others.

Despite all these opening policies, none of these countries has managed to implement an effective method to completely eliminate or reduce to a minimum the entire parallel market to the legal/regulated one. For this reason, it is necessary to develop new tools for the different actors involved in the legalization/regulation of cannabis, either from a "recreational" or "medical" perspective.

One of these tools can be the creation of methods to correctly identify the source of a given sample of *Cannabis.* This could establish whether a given sample is from illegal origin, or from a variety that is legally traded.

Current methods can differentiate cannabis samples from other substances, but they are not specific enough to distinguish samples of cannabis from each other and to conclude whether another sample found later comes from the first variety tested, i.e. they are not useful for sample traceability.

DNA-only identification analyses are complicated in the case of plants, because for a sample from the same place of origin, there may be cross-breeding, pollination, climate, etc. and therefore, the genetic profile may be somewhat different from what is expected or known from the database^{5,6}.

Furthermore, the chemical composition of a plant is derived from its specific environment. The chemical methods used to identify the samples and the origin of a cannabis sample include palynology, the study of the composition in spores and pollen⁷, the obtaining of the sample chemical profile⁸, or isotope analysis and the ratio of isotopes in samples, which are analyzed by mass spectrometry^{9,10}.

On the other hand, the genetic methods found in the literature are based on *Cannabis* DNA amplification^{11,12,13,14}, which has allowed forensic investigators to move away from the identification mentioned in the previous paragraph. These documents describe the use of short tandem repeats (STRs), single sequence repeats (SSRs) or microsatellites for the determination of a single DNA strand.

Document US2006/0035236 A1 discloses the use of STRs in multiplex DNA analysis for Cannabis samples determination¹⁵. However, sample prior separation in a polyacrylamide gel is required. All these analyses involve pre-treatment of the sample, which usually leads to decarboxylation of the terpenic derivatives and can result in loss of information and misidentification of a sample.

In the literature, information on the determination of the chemical composition of this plant has only been found by NMR techniques, either using two-dimensional NMR-¹H techniques¹⁶ and the combined use of NMR-¹H with HPLC, analyzed on the sample, after separation by chromatographic techniques¹⁷. However, in the present invention a ¹³C spectrum is also made, whose advantage is that there is a greater resolution between observed peaks, almost without signal overlapping. Signal overlapping is a common disadvantage in NMR-¹H which leads to an increasing difficulty of the analysis. In addition, a greater range of frequencies can be analyzed, making the analysis more reliable.

What all the methods mentioned in the previous paragraphs have in common, regardless of the method used, is that their objective is the identification of cannabis samples with respect to other substances. However, the present invention is an improvement, as it allows the reliable and rapid identification of cannabis samples varieties with respect to other cannabis samples by means of fingerprint profiles.

The object of this invention is a rapid and standardized method that comprises molecular analysis techniques of DNA markers and the determination of the resonance pattern in NMR¹³C and ¹H, to obtain its fingerprint and thus determine its origin and traceability with respect to other cannabis samples. This allows the sample to be traced and distribution lines identified, achieving a degree of reliability in identification that exceeds that of any current analysis.

The method of the present invention has the following advantages:
- it provides valuable information concerning the propagation and distribution of cannabis from growers, processors, transporters or vendors involved in its distribution chain,
- allows to detect the deviation of the cannabis product through a parallel channel to the legally established one, either intra or extra border,
- allows the detection of unauthorized marketing of registered/legal varieties,
- certification of the origin and verification of the health standards of the specified cannabis product,
- determination of the origin of a dubious sample and its categorization according to the legislation in force,
- verification of the traceability by fingerprinting of the material produced by both the licensees and the producers of the illegal/unregulated market and inviolability of the results obtained,
- provide the necessary information in advance of the characteristics of any cannabis product subject to patent/license,
- Objective demonstration and scientific certainty of the regulatory model of cannabis developed by any country that has incorporated the method of the invention,
- Databases creation from analyzed samples to facilitate police and legal work,
- to provide police agencies with the ability to link all offenders related to the crime, to bring the whole chain of crime involved to the surface; and
- linking transnational cannabis trafficking investigations.

### DESCRIPTION OF THE INVENTION

The present invention refers to a method of obtaining a fingerprint of cannabis samples that comprises the following steps:
a) determination of the genetic profile by the analysis of at least one STR marker
b) obtaining the chemical profile by means of NMR; and
c) differentiation of cannabis varieties from each other by comparing the results obtained in steps (a) and (b) for each sample with each other and/or with databases of previously obtained fingerprints.

The term "sample" or "plant sample" in the present invention refers to any part of the cannabis plant, preferably leaves, stem, bulb, seeds or flowers, more preferably leaves. Such plant sample weighs between 0.1 and 10 g, preferably 0.2 and 1.0 g, more preferably 0.5 g.

The term "fingerprint" in the present invention refers to a combination of particular genetic and chemical characteristics which enable the identification and traceability of a sample of cannabis and differentiate it from other varieties of the same species.

Additionally, the method of the invention can differentiate plant samples from individuals of the same variety from other individuals of the same variety who have been subjected to particular and different environmental conditions.

The term "multiplex" is the ability to perform multiple simultaneous analyses in a single trial, the procedure for implementing such an ability in a process is called a "multiplexed trial". It is applied in procedures that analyze several loci simultaneously.

The terms "markers" and "microsatellite markers" have equivalent meanings, referring to DNA sequences containing repetitive fragments, and are used interchangeably in the present invention.

Any other term used in the present specification shall have the usual meaning in the field of technology to which the present invention refers.

In the method of the invention the steps a) and b), and according to claim 1, do not have to be carried out in the order indicated. That is to say, it is not a chronological order, they can be carried out in the mentioned order or vice versa.

The STR analyzed in the method of the present invention are distributed throughout the genome and can preferably be selected among the following STR: D02, C11, H09, B01, E07, 305, 308, B05, H06, 501, CS1, 302, 301, B02, H11 and combinations thereof. Table 1 shows the GenBank database access numbers:

**TABLE 1: Identification of STR genetic markers.**

| **Marker** | **Access number** |
|---|---|
| D02 | KT203591 |
| C11 | KT203583 |
| H09 | KT203598 |
| B01 | KT203579 |
| E07 | KT203593 |
| 305 | KT203571 |
| 308 | KT203574 |
| B05 | KT203581 |
| H06 | KT203596 |
| 501 | KT203577 |
| CS1 | KT203586 |
| 302 | KT203569 |
| 301 | KT203566 |
| B02 | KT203580 |
| H11 | KT203601 |

The step of determining the genetic profile of the cannabis sample from at least one STR comprises the following stages:
- extraction and quantification of DNA from the plant sample, and
- amplification of the selected STR by individual PCRs or multiplexed PCRs and analysis of the PCR results.

These steps can be performed by any means known in the state of the art, preferably by multiplexed PCR or individual marker PCR.

Preferably, the results of individual PCRs can be analyzed by agarose gels and the results of multiplexed PCR by capillary electrophoresis.

A further realization of the invention involves the pre-freezing at a temperature between -4°C to -80°C of the sample from which the STR are to be determined until its extraction.

A further embodiment of the invention comprises the detection of the presence of the Rubisco gene in cannabis samples prior to the determination of the genetic profile by STR marker analysis to confirm that the sample is a plant sample.

The presence of the Rubisco gene can be checked by sequencing and with any known state-of-the-art method involving amplification, Sanger sequencing and comparison of results with cannabis from public data bases.

Obtaining the NMR chemical profile of the cannabis sample involves obtaining the ¹H and ¹³C spectrum.

The NMR technique allows the analysis of the content of secondary metabolites, mainly cannabinoids and terpenes.

Obtaining the NMR chemical profile of the cannabis sample involves the following steps: extraction, dissolution in deuterated solvent, obtaining and analyzing the ¹H and ¹³C spectrum.

The sample extraction is performed with a solvent selected from among:
- an alcohol from 1 to 6 carbons,
- one alkane from 1 to 6 carbons and
- an alkane halide derivative,
preferably ethanol and methanol, more preferably ethanol, for a period of time comprised between 8 and 24 hours, preferably 12 hours, and a temperature between 15 and 30°C, preferably 25°C.

Additionally, the deuterated solvent is selected from the deuterated variants of: chloroform, acetone, methanol, benzene, heavy water, dimethyl sulfoxide, dichloromethane, trifluoroacetic acid, acetonitrile, pyridine, *N,N*-dimethylformamide and tetrahydrofuran, preferably chloroform.

Sample processing for NMR analysis involves the use of a chemical drying agent, selected from anhydrous magnesium sulfate, sodium sulfate and calcium chloride.

The determination can be performed based on the signal pattern in the ¹H-NMR spectrum that appears between:
- 0.0 to 0.8 ppm, assigned to the lipidic composition of the sample, of esterified fatty acids,
- signals that appear from 0.6 to 3 ppm preferably 0.8 to 2.5 ppm, corresponding to the aliphatic protons of the terpenic fraction, and
- the signals that appear from 3.2 to 7 ppm, preferably 3.8 to 6.5 ppm, which are assigned to protons on unsaturated carbons of the same fraction.

The integration ratio of the zones of the different aliphatic protons versus unsaturated ones is comprised in the range: 20:8 to 40:8, preferably 30:8, and corresponds to the chemical composition present in the extract.

In an additional embodiment, the ¹H spectrum is confirmed by analysis of signals of ¹³C-NMR spectrum acquired from 0 to 180 ppm. There is a correspondence between signals corresponding to the aliphatic, unsaturated and aromatic carbon atoms with those found in ¹H-NMR spectrum.

¹³C-NMR spectrum is analyzed in the range from 100 to 180 ppm, preferably the range from 160 to 180 ppm. In this area there are signals corresponding to carbon atoms assigned to carbonyl groups that belong to the terpenic fraction and which have not undergone decarboxylation in the extraction process, mainly due to the absence of very high temperatures in said stage.

¹³C-NMR spectrum analysis comprises at least 40 frequencies (ppm) of peaks associated with metabolites present in the sample, more preferably at least 60 frequencies.

The agreement between STR and NMR patterns obtained through the analyses, as well as their reproducibility, increases the degree of certainty in the identification of cannabis samples to values that have not been achieved so far. Until now, legal analysis of cannabis samples was generally limited to determining the net weight of the sample and analyzing the THC percentage, and therefore the combination of NMR and STR techniques has a clear advantage over the state of the art. It can be concluded that the combination of both methods allows a more accurate identification with less error than any other method known so far.

Samples can be from plants of the genus *Cannabis* and are selected from *Cannabis sativa* L. *sativa* and *indica* subspecies, *sativa* variety; *Vavilov* spontaneous variety; *indica* (Lam.) variety, *kafiristanica* (Vavilov) variety and combinations thereof, more preferably *Cannabis sativa* L.

The sample of the cannabis plant is selected from leaves, stem, bulb, seeds and flowers, preferably leaves.

The sample amount used to obtain the fingerprint is from 0.1 to 10 g, preferably 0.2 to 1.0 g, more preferably 0.5 g.

An additionalobject of the invention is the creation of a database comprising the fingerprints of samples of cannabis obtained by the method described above.

Such a database will enable the fingerprint of a sample to be compared with all previously obtained fingerprints stored in that database.

The present invention is illustrated by the following examples which are not intended to be limiting.

### BRIEF DESCRIPTION OF THE FIGURES

**FIGURE 1****:** Agarose gel showing the amplified Rubisco gene fragment from sample No 680 in triplicate, "M" being a molecular weight marker, "607" a positive control and "C-" a negative control.
**FIGURE 2****:** Screenshot of the Rubisco gene nucleotide sequence comparison results obtained from the cannabis sample No 680 with the NCBI (National Center for Biotechnology Information) database. At the bottom it is noted that the results obtained are from the *Cannabis* genome.
**FIGURE 3****:** Electropherogram of the STR D02 marker alleles in the 5 cannabis samples analyzed (680, 681, 682, 683 and 684).
**FIGURE 4****:** Electropherogram of the STR C11 marker alleles in the 5 cannabis samples analyzed (680, 681, 682, 683 and 684).
**FIGURE 5****:** Electropherogram of the STR H09 marker alleles in the 5 cannabis samples analyzed (680, 681, 682, 683 and 684).
**FIGURE 6****:** Electropherogram of the STR B01 marker alleles in the 5 cannabis samples analyzed (680, 681, 682, 683 and 684).
**FIGURE 7****:** Electropherogram of the STR E07 marker alleles in the 5 cannabis samples analyzed (680, 681, 682, 683 and 684).
**FIGURE 8****:** Electropherogram of the STR 305 marker alleles in the 5 cannabis samples analyzed (680, 681, 682, 683 and 684).
**FIGURE 9****:** Electropherogram of the STR 308 marker alleles in the 5 cannabis samples analyzed (680, 681, 682, 683 and 684).
**FIGURE 10****:** Electropherogram of the STR B05 marker alleles in the 5 cannabis samples analyzed (680, 681, 682, 683 and 684).
**FIGURE 11****:** Electropherogram of the STR H06 marker alleles in the 5 cannabis samples analyzed (680, 681, 682, 683 and 684).
**FIGURE 12****:** Electropherogram of the STR 501 marker alleles in the 5 cannabis samples analyzed (680, 681, 682, 683 and 684).
**FIGURE 13****:** Electropherogram of the STR CS1 marker alleles in the 5 cannabis samples analyzed (680, 681, 682, 683 and 684).
**FIGURE 14****:** Electropherogram of the STR 302 marker alleles in the 5 cannabis samples analyzed (680, 681, 682, 683 and 684).
**FIGURE 15****:** Electropherogram of the STR 301 marker alleles in the 5 cannabis samples analyzed (680, 681, 682, 683 and 684).
**FIGURE 16****:** ¹H NMR spectrum of cannabis sample No 680.
**FIGURE 17****:** ¹³C NMR spectrum of cannabis sample No 680 (top image), magnification of a region of the spectrum (lower image).
**FIGURE 18****:** ¹H NMR spectrum of cannabis sample No 681.
**FIGURE 19****:** ¹³C NMR spectrum of cannabis sample No 681 (top part), magnification of a region of the spectrum (bottom part)
**FIGURE 20****:** ¹H NMR spectrum of cannabis sample No 682.
**FIGURE 21****:** ¹³C NMR spectrum of cannabis sample No 682.
**FIGURE 22****:** ¹H NMR spectrum of cannabis sample No 683.
**FIGURE 23****:** ¹³C NMR spectrum of cannabis sample No 683.
**FIGURE 24****:** ¹H NMR spectrum of cannabis sample No 684.
**FIGURE 25****:** ¹³C NMR spectrum of cannabis sample No 681 (top part), magnification of a region of the spectrum (bottom part).

### EXAMPLES

The analyses carried out have been made with leaf samples from 5 *Cannabis Sativa* L varieties as shown in Table 2.

**TABLE 2: Samples used in the experiments**

| SAMPLE NUMBER | AMOUNT/UNIT |
|---|---|
| 680 | 1.6 g x 4 Units |
| 681 | 1.4 g x 3 Units |
| 682 | 1.7 g x 3 Units |
| 683 | 7 g x 1 Units |
| 684 | 1.5 g x 3 Units |

### Molecular analysis of DNA markers

### DNA Extraction

For the genetic sequencing analysis, the samples were kept frozen at a temperature of -20°C from reception until the analysis. The sample is then cut into small portions using sterile blades and homogenized with liquid nitrogen. DNA extraction has been carried out using the method of cethylmethylammonium bromide (CTAB) in water on the samples, following a manual and standard forensic extraction method¹⁸. The main steps are described below:
The sample is ground with liquid nitrogen in a mortar to obtain a fine powder. Then, 300 µl of extraction buffer (0.1 mM EDTA, 10 mM Tris-HCI pH 7.5) with 0.2% betamercaptoethanol are added. is the resulting product is mixed and incubated for 1 hour at 60 °C in a shaker. Then, 300 µl of isoamyl alcohol chloroform (24:1) are added and mixed by inversion. Centrifugation for 10 minutes at 14500 rpm is carried out and transfer of the aqueous phase into a clean tube, to which 1 volume (about 200 µl) of isopropanol is added, mixed by inversion and the samples are incubated for 10 minutes at room temperature. Then centrifugation of the tubes for 10 minutes at 10000 rpm is carried out, discarding the supernatant, washing the pellet obtained with 300 µl 70% ethanol and centrifugation of the tubes for 5 minutes at 10000 rpm, discarding the supernatant again and allowing the pellet to dry. Finally, the sample is redissolved in 50 µl of double-distilled, nuclease free water and the purified DNA is quantified. DNA quantification and quality analysis (A280/230 and A230/A260) were obtained by a spectrophotometric method, using the NanoVue spectrophotometer. Each sample was analyzed in triplicate.

### Sequencing the Rubisco gene

The genetic confirmation of the species (Barcoding) was obtained by PCR amplification and Sanger sequencing of the Rubisco gene fragment. The sequence obtained was searched in the international GenBank database.

The amplified Rubisco gene fragment is the one delimited by the design of the primers published in the scientific article by Kress et al.²¹ The size of the amplification fragment generated with these primers is about 700 base pairs.

The PCR conditions were as follows: 98°C - 30 seconds, 40 cycles (98°C - 10 seconds; 52°C - 30 seconds; 72°C - 30 seconds), 72°C - 2 minutes.

The reagents, reaction volume and controls are as follows
Reagents kit used: New England Biolab (NEB) Q5 High-fidelity DNA polymerase M0491S.
- Control samples. External control: DNA extracted from oleander leaf, Nerium Oleander. Negative control: distilled water.
- The reaction volumes are shown in Table 3:

**TABLE 3: Reaction volumen**

| **Reagents** | **per sample (µl)** |
|---|---|
| Water | 11.3 |
| PCR buffer | 5 |
| Q5 GC (Kit specific) | 5 |
| dNTPs 10 mM | 0,5 |
| primers 10 µM (each) | 1 |
| Q5 High-fidelity Taq | 0,2 |
| | |
| Total mixed volume per reaction | 24 µl |
| Total DNA in the reaction | 1-100 ng |

The amplified gene fragment was analyzed in a 2% agarose gel.

For the analysis of the PCR results an agarose gel (2%) was prepared: Agarose D1 LE (Low EEOO) Cat. No E5000 INtRON biotechonology: 0.7 g. TBE 1X buffer: 35 ml. Sybr safe DNA gel stain. Invitrogen Ref S33102: 3,5 µl. The molecular weight marker used was the Omega M01-02 100 bp DNA ladder. Promega blue/Orange loading buffer 6x Ref: G190A was added to the samples before loading them into the gel. A volume of 3-5 µl of loaded PCR/marker product was loaded into each well. The electrophoresis conditions were 90 Watt for a period of 30-45 min.

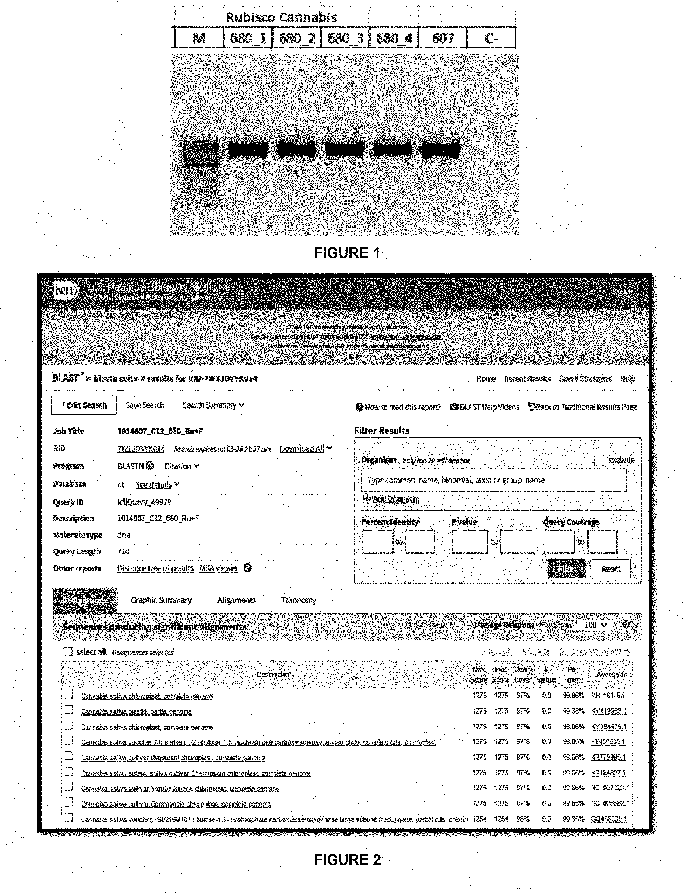

FIGURE 1 shows the result obtained from sample 680. The above protocol was repeated for the remaining samples (681-684) with identical results.

A Sanger sequencing of the samples was additionally performed using the same primers¹⁹. The nucleotide sequences obtained were searched into the NCBI open access database, confirming that the amplified fragment corresponds to the Rubisco gene, FIGURE 2.

### Analysis of STR molecular markers

A total of 13 pairs of previously published primers were used^{20,21} for the amplification of the microsatellites detailed in table 4. For capillary electrophoresis, the Forward primer of each pair was marked with a fluorophore for subsequent separation and analysis²¹:

**TABLE 4: Primers pairs used in the molecular analysis of DNA markers in cannabis samples**

| **STR** | **primer FORWARD (5'-3')** | **primer REVERSE (5'-3')** | **fluorophore²¹** |
|---|---|---|---|
| **D02** | GGTTGGGATGTTGTTGTTGTG | AGAAATCCAAGGTCCTGATGG | 6-FAM™ |
| **C11** | GTGGTGGTGATGATGATAATGG | TGAATTGGTTACGATGGCG | 6-FAM™ |
| **H09** | CGTACAGTGATCGTAGTTGAG | ACACATACAGAGAGAGCCC | 6-FAM™ |
| **B01** | TGGAGTCAAATGAAAGGGAAC | CCATAGCATTATCCCACTCAAG | 6-FAM™ |
| **E07** | CAAATGCCACACCACCTTC | GTGGTAGCCAGGTATAGGTAG | VIC® |
| **305** | AAAGTTGGTCTGAGAAGCAAT | CCTAGGAACTTTCGACAACA | VIC® |
| **308** | AGATGGTGTTGGGTATCTTT | TGGTGCAGGTTTATACAATTT | VIC® |
| **B05** | TTGATGGTGGTGAAACGGC | CCCCAATCTCAATCTCAACCC | VIC® |
| **H06** | TGGTTTCAGTGGTCCTCTC | ACGTGAGTGATGACACGAG | VIC® |
| **501** | AGCAATAATGGAGTGAGTGAAC | AGAGATCAAGAAATTGAGATTCC | NED™ |
| **CS1** | AAGCAACTCCAATTCCAGCC | TAATGATGAGACGAGTGAGAACG | NED™ |
| **302** | AACATAAACACCAACAACTGC | ATGGTTGATGTTTTGATGGT | PET™ |
| **301** | ATATGGTTGAAATCCATTGC | TAACAAAGTTTCGTGAGGGT | PET™ |

and for the analyses a PCR thermocycler, a gel documenter and a genetic analyser have been used. The genetic method has been developed by amplification of STRs microsatellite markers through multiplexed PCR, using the techniques previously indicated.

### Multiplexed PCR (13 markers):

PCR program: 98°C 30s; 7 cycles (98°C 10s; 61°C 30s, 72°C 30s); 5 cycles for each temperature (98°C 10s; *touchdown** °C, 30s, 72°C 30s); 7 cycles (98°C 10s; 51°C 30s, 72°C 30s); 72 °C 2 min. *Touchdown*:* 60, 59, 57, 54, 52 °C

The concentration of primers in each reaction is shown in Table 5:

**TABLE 5: Concentration of primers used in each reaction**

| **Marker** | **µM** |
|---|---|
| **D02** | 0,08 |
| C11 | 0,08 |
| **H09** | 0,16 |
| **B01** | 0,09 |
| **E07** | 0,16 |
| **305** | 0,12 |
| **308** | 0,26 |
| **B05** | 0,03 |
| **H06** | 0,07 |
| **501** | 0,1 |
| **CS1** | 0,14 |
| **302** | 0,16 |
| **301** | 0,4 |

The reagents, reaction volume and controls are
- Reagent kit used: New England Biolab (NEB) Q5 High-fidelity DNA polymerase M0491S.
- Negative control sample: distilled water.
- The reaction volume is shown in Table 6:

**TABLE 6: Reaction volume**

| **Reagents** | **Per sample (µl)** |
|---|---|
| Water | 10,30 |
| PCR Buffer | 5 |
| Q5 GC (Kit specific) | 5 |
| dNTPs 10 mM | 0,5 |
| primers (mix) | 3 |
| Q5 High-fidelity Taq | 0,2 |
| | |
| Total mixed volume per reaction | 24 µl |
| Total DNA in the reaction | 25 ng aprox. |

### Capillary electrophoresis and fragment analysis

Fragment separation and detection of PCR products was performed in Genetic Analyzer 3130 (Applied Biosystems).

1-2 µL of each PCR product were loaded to a 10 µL mixture (9.5 µl of "Hi-Di Formamide®" and 0.5 µl of the LIZ® 500 molecular weight marker. The samples were denatured 5 minutes at 94°C before being loaded into the Genetic Analyzer 3130, and run under the following conditions: Oven at 60°C; prerun 15 kV; injection 1.2 kV, 16 s; run 15 kV, 1200 s; capillary length 36 cm; polymer: POP-7; Dye Set G5 probe set.

Simultaneously to the analysis of the fragments by capillary electrophoresis, a 2% agarose gel can be made according to the protocol indicated above, and part of the multiplex PCR product can be run therein to determine that the amplification occurred correctly.

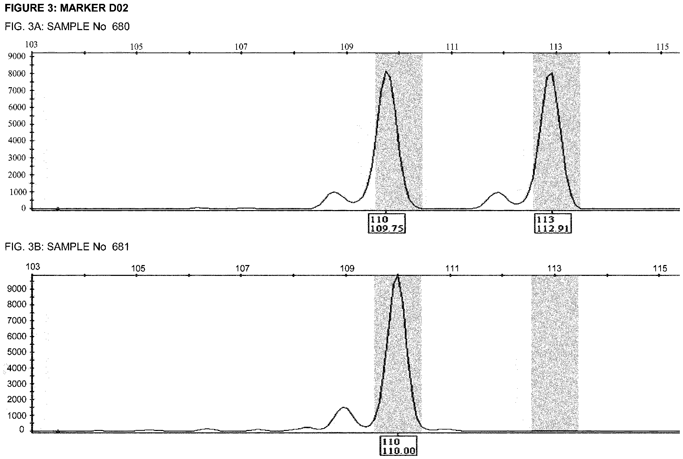

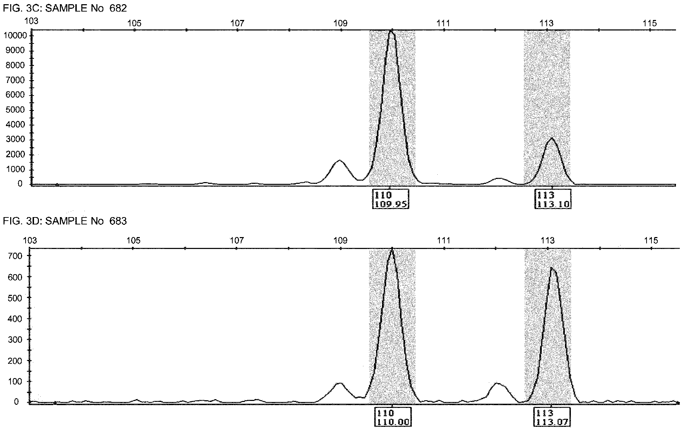

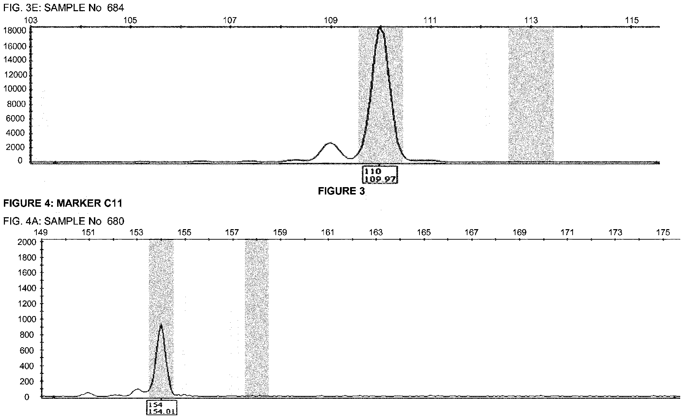

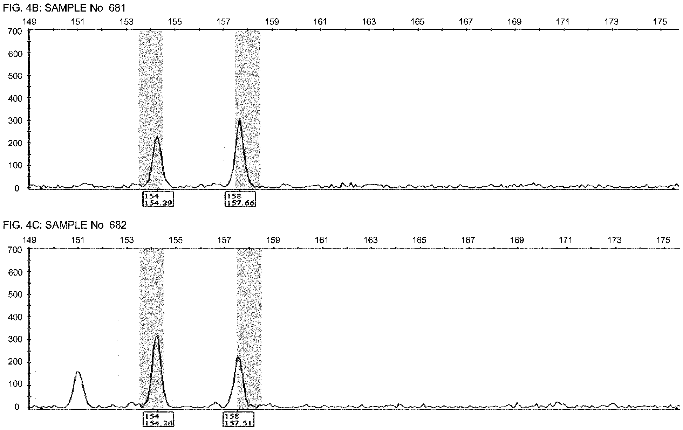

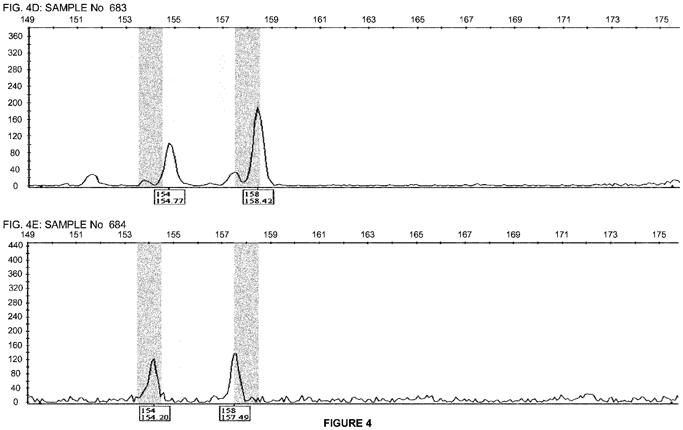

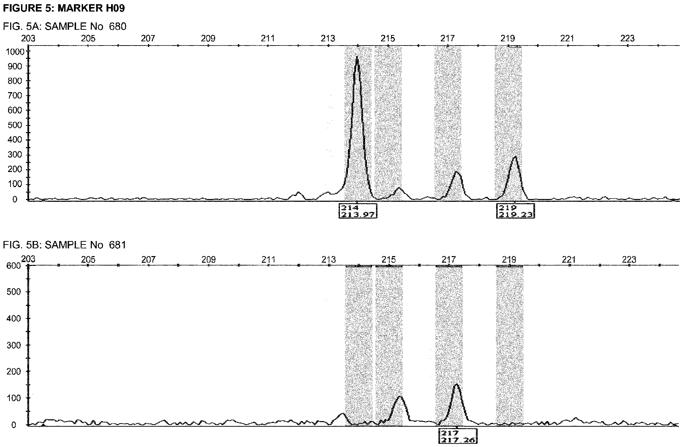

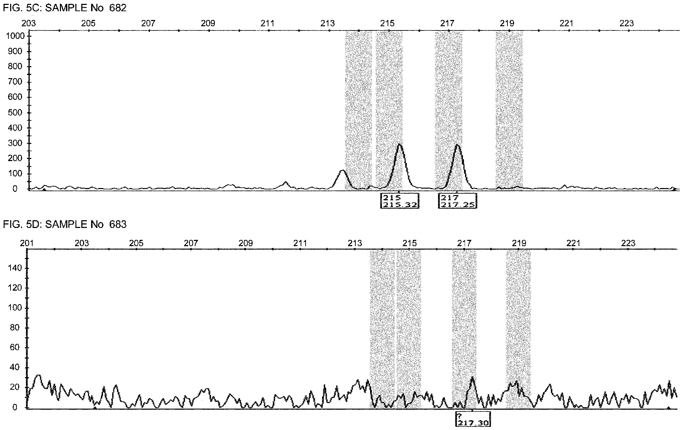

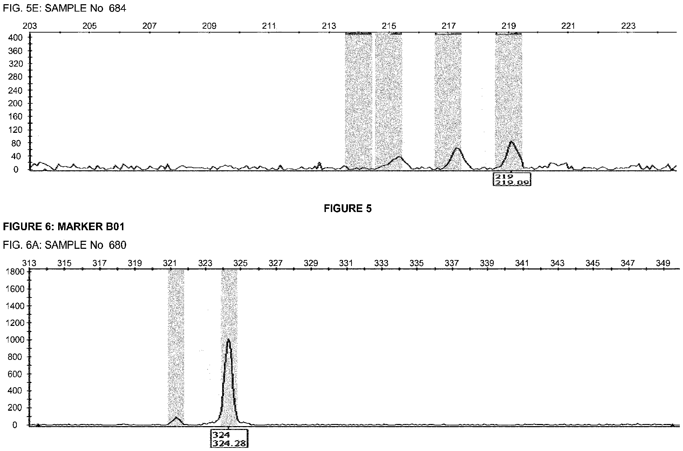

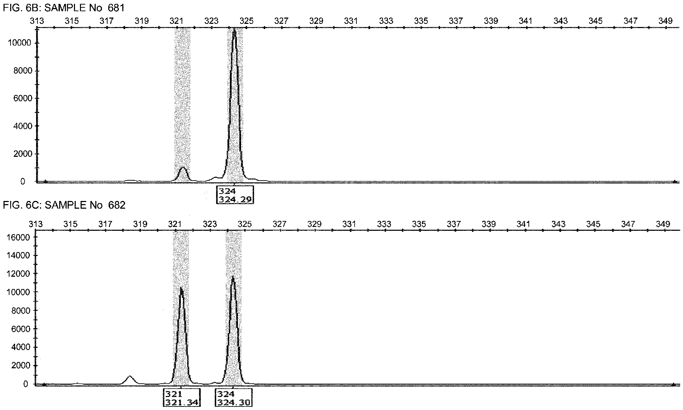

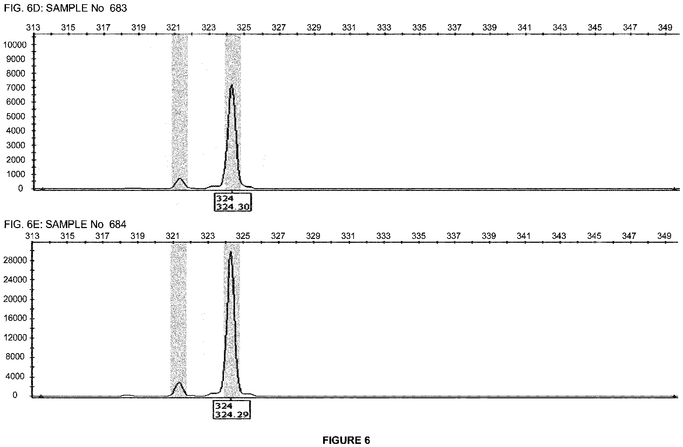

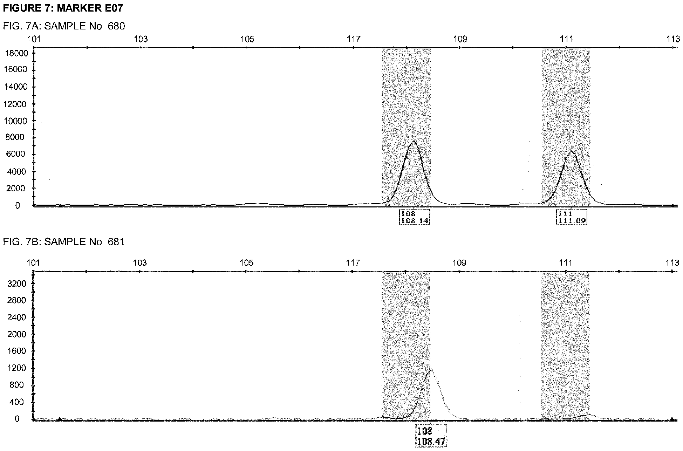

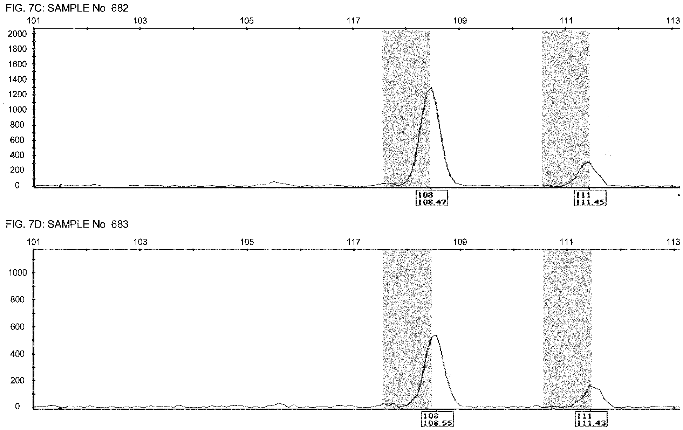

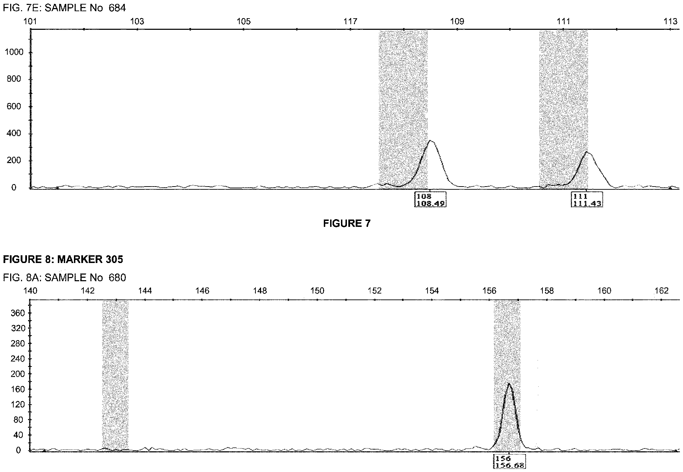

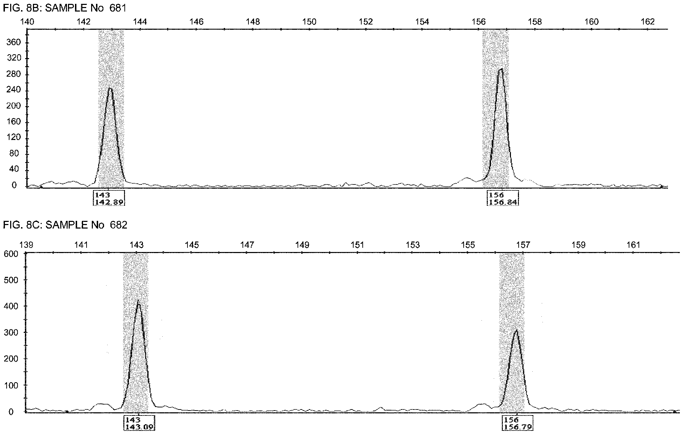

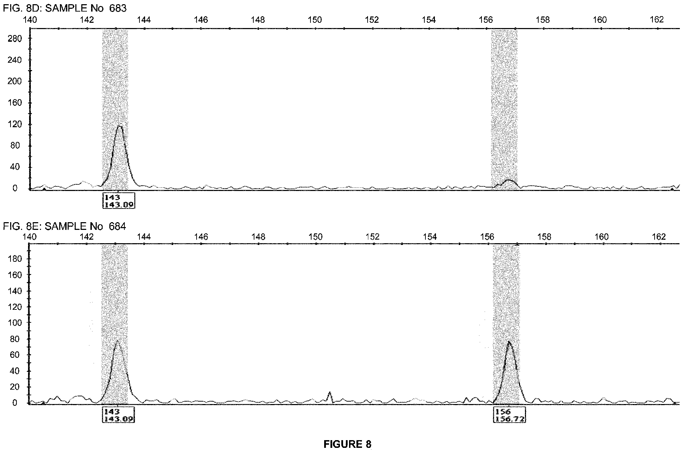

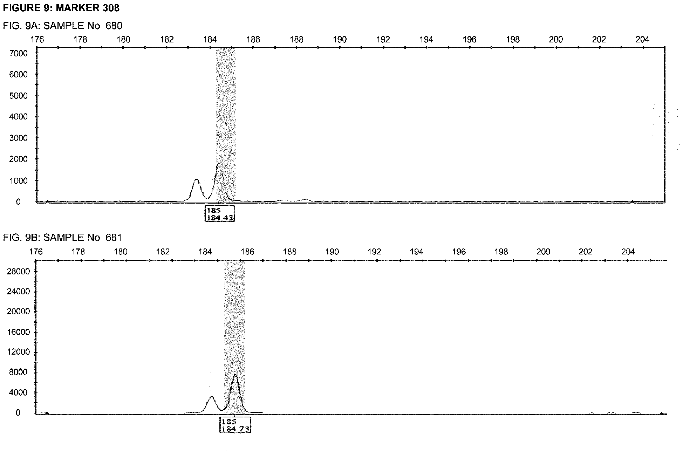

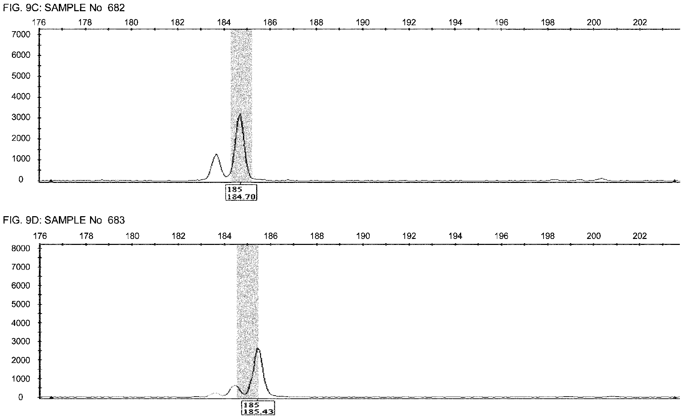

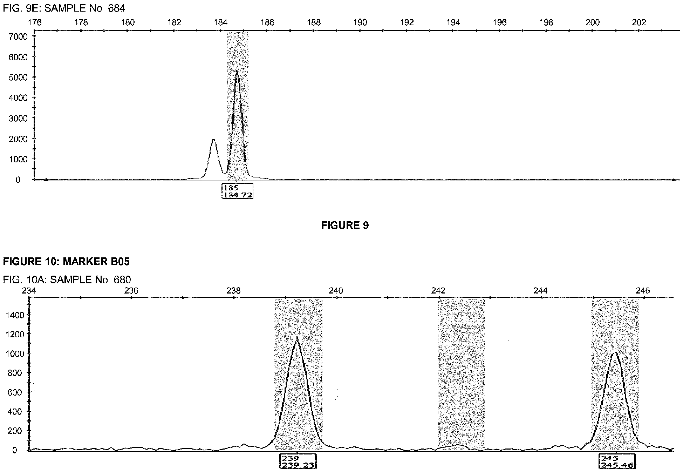

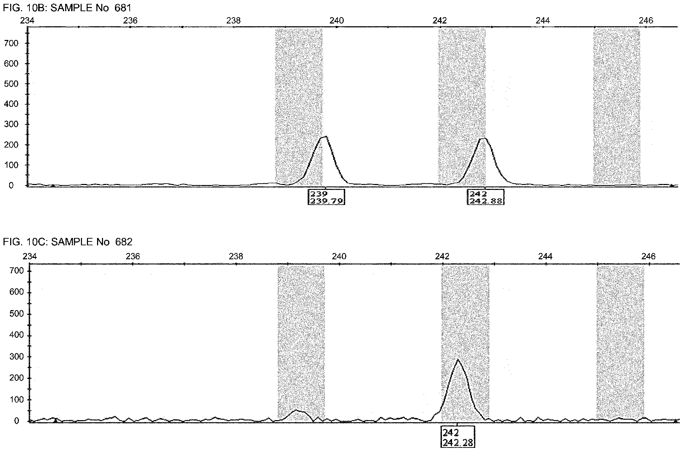

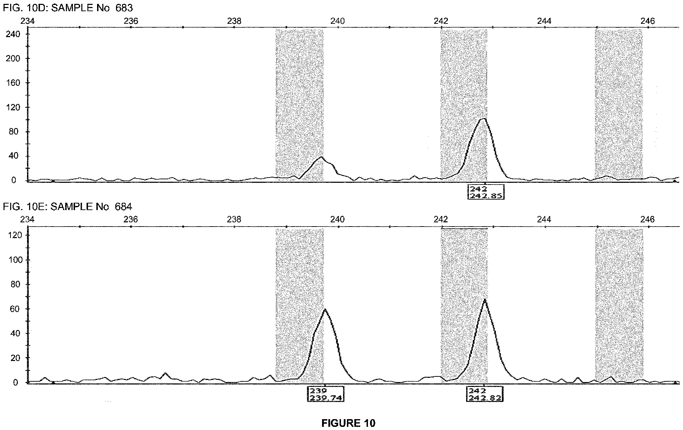

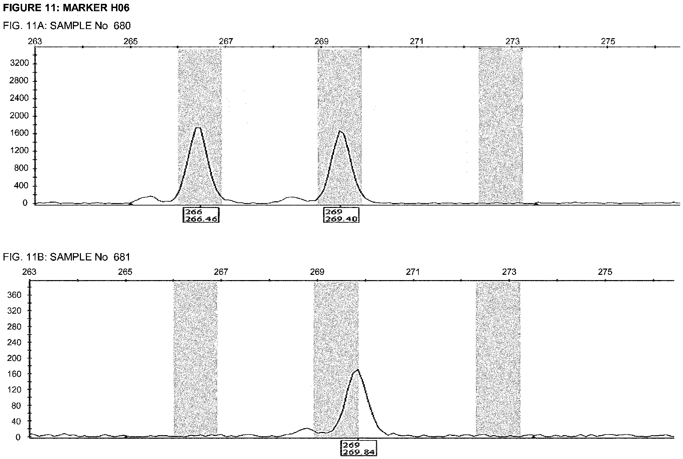

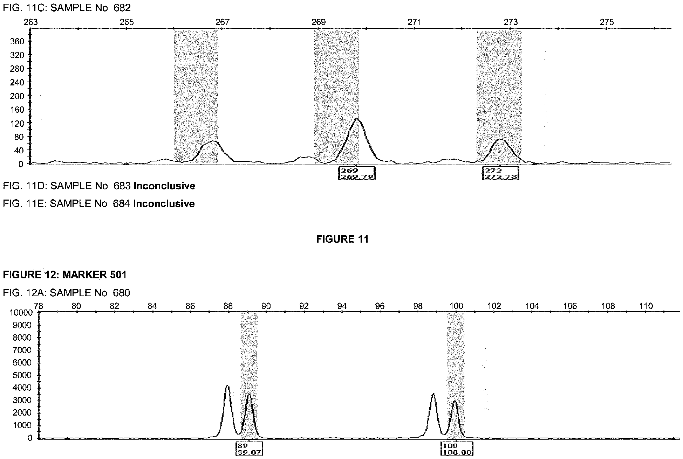

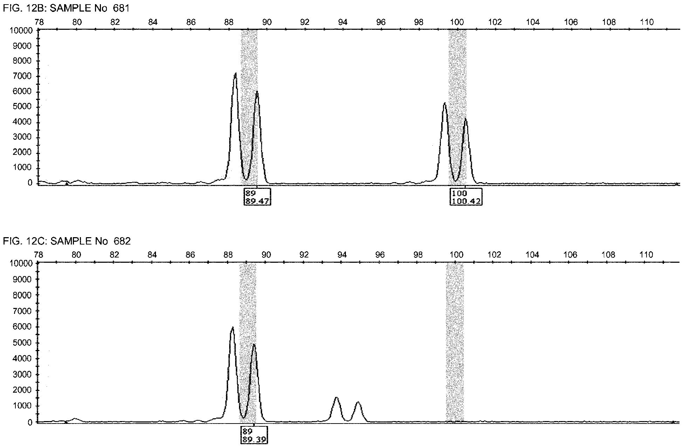

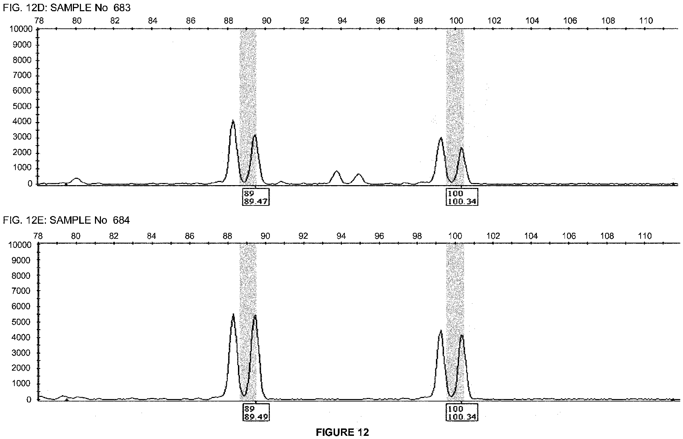

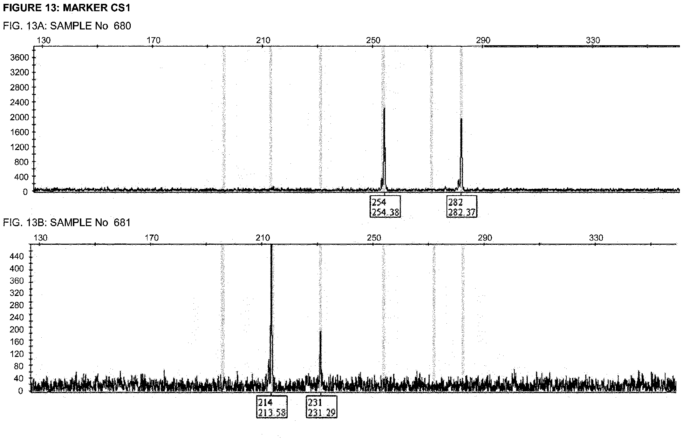

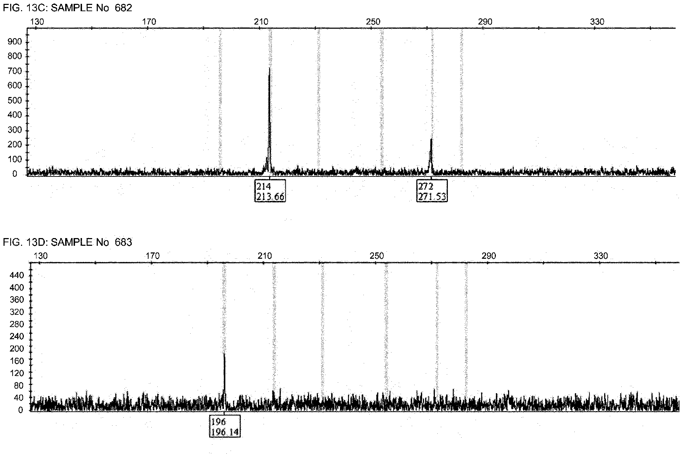

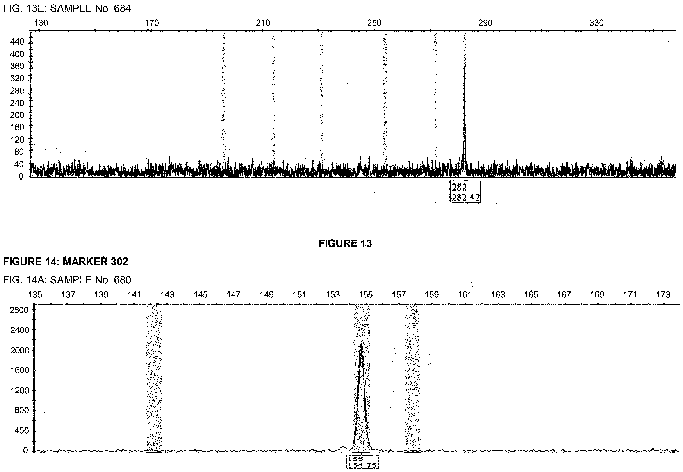

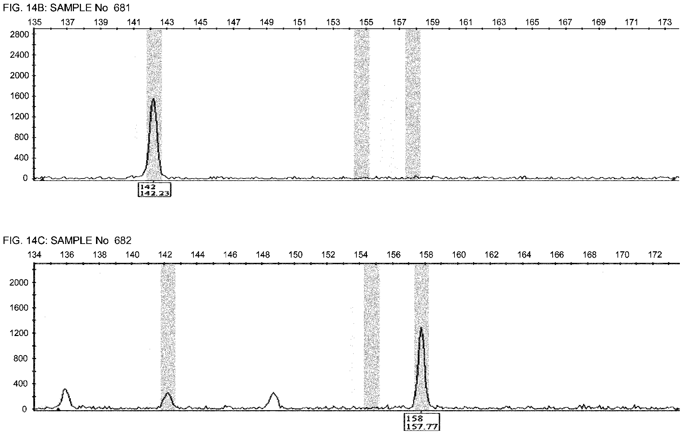

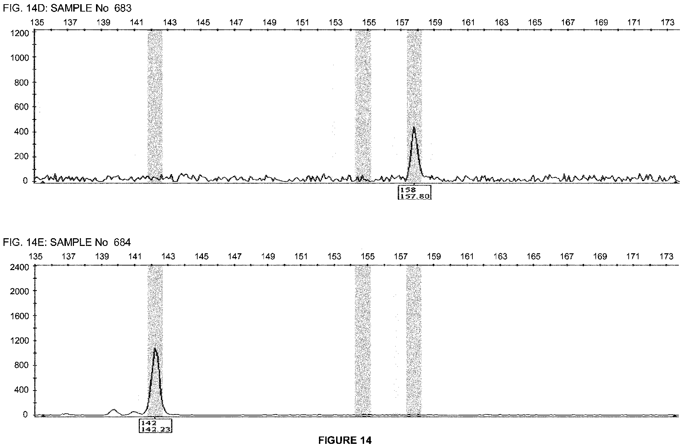

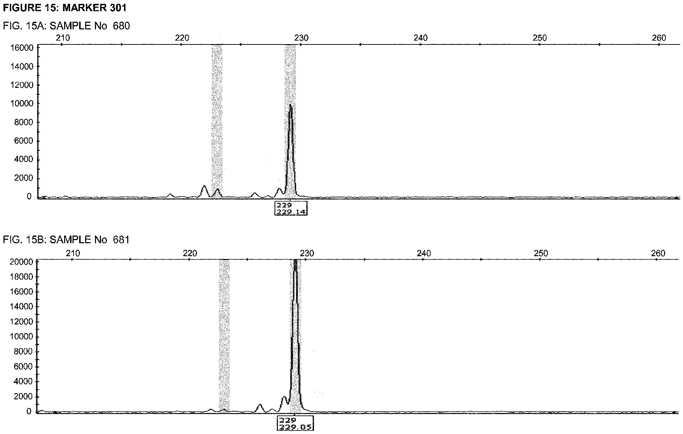

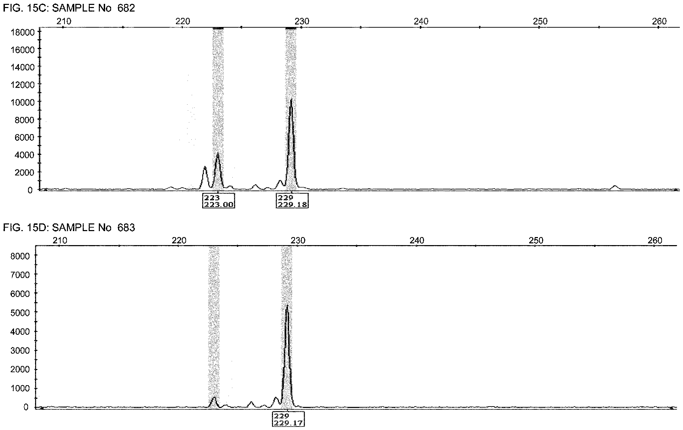

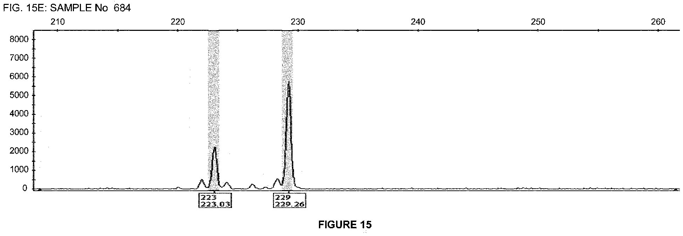

The analysis of results was performed with GeneMapper® v5.0 Software (Applied Biosystems). They can be seen in figures 3-15. The grey colored area in the figures represents a different allele, the software allows to "write down" this allele, memorizing the detection of this allele in subsequent analyses of different samples. The grey areas represent the size of the alleles detected either in the sample being analyzed, or in other samples that were analyzed previously.

Table 7 shows the results obtained for the STR marker alleles (columns) in the samples tested (rows).

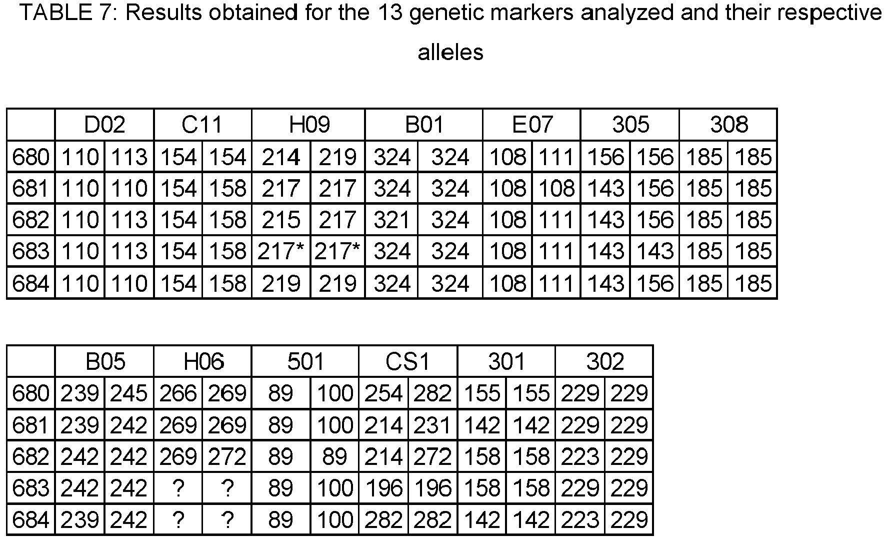

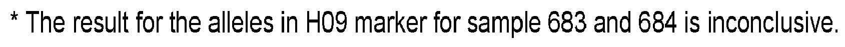

Repeated values have been found in the patterns of the markers and each of the markers can be assigned to the samples analyzed. Table 8 shows the number of identical STR markers in each comparison and table 9 shows the % similarity between the samples.

**TABLE 8: Number of identical STR markers between cannabis samples**

| | | | | | |
|---|---|---|---|---|---|
| | 680 | 681 | 682 | 683 | 684 |
| 680 | | 4 (13) | 3(13) | 6 (12) | 4 (12) |
| 681 | | | 3(13) | 6 (12) | 8 (12) |
| 682 | | | | 5 (12) | 5 (12) |
| 683 | | | | | 5 (12) |
| 684 | | | | | |

**TABLE 9: Percentage of similarity between cannabis samples**

| | | | | | |
|---|---|---|---|---|---|
| | 680 | 681 | 682 | 683 | 684 |
| 680 | | 30,77 | 23,08 | 50,00 | 33,33 |
| 681 | | | 23,08 | 50,00 | 66,67 |
| 682 | | | | 41,67 | 41,67 |
| 683 | | | | | 41,67 |
| 684 | | | | | |

Although all samples have a different marker pattern, the degree of similarity between the 681 and 684 samples (8 identical markers, 66.67%) is so high that it cannot be said with sufficient certainty that these samples do not have the same origin. This is why the determination of phenotypic characteristics by means of the ¹H and ¹³C spectra provides key information to obtain the fingerprint of each cannabis sample in order to determine its traceability.

### Nuclear Magnetic Resonance

### Optimization of the extraction step

In order to obtain the corresponding NMR spectra it is necessary to extract the metabolites present in the plant, a process that required previous optimization. For this purpose, a Soxhlet extraction equipment and different solvents (ethanol, isopropanol, dichloromethane, chloroform or hexane) were used for the extraction and at laboratory scale.

However, it was found that the extraction processes using a Soxhlet equipment, which in practice take place under heat and at temperatures close to the boiling point of the extraction solvent, lead to decarboxylation of cannabinoids into acidic forms. For this reason, it was decided to carry out the extraction at room temperature, thus preserving the acidic derivatives of cannabinoids. In this "cold" extraction (which includes values between 10 and 25 °C), the samples were previously lyophilized and, after being powdered,, they were subjected to extraction with different solvents (ethanol, isopropanol, dichloromethane, chloroform, acetonitrile, tetrahydrofuran or hexane) during different times (8, 12, 24 hours). This study made it possible to establish the optimum conditions for the extraction by weight of the extract and the analysis of the cannabinoid content by means of a gas chromatography (GC) or liquid chromatography (HPLC) technique. The results are summarised in Table 10:

**TABLE 10: Optimisation of cannabinoids extraction**

| **Test** | **Solvent** | **Cycles/ time** | **Conditions** | **Yield¹** | **Composition²** |
|---|---|---|---|---|---|
| 1 | Ethanol | 1 | Reflux (Soxhlet) | 32.2% | 20.1% |
| 2 | Ethanol | 2 | Reflux (Soxhlet) | 32.5% | 20.4% |
| 3 | Ethanol | 3 | Reflux (Soxhlet) | 32.7% | 20.2% |
| 4 | Isopropanol | 2-3 | Reflux (Soxhlet) | 18-21% | 18.7% |
| 5 | Hexane | 2-3 | Reflux (Soxhlet) | 10-12% | 25.5% |
| 6 | CH₂Cl₂ | 2-3 | Reflux (Soxhlet) | 21-23% | 20.8% |
| 7 | CHCl₃ | 2-3 | Reflux (Soxhlet) | 19-24% | 21.2% |
| 8 | Ethanol | 8 hours | 25 °C | 27.5% | 23.5% |
| 9 | Ethanol | 12 hours | 25 °C | 27.8% | 23.5% |
| 10 | Ethanol | 24 hours | 25 °C | 27.9% | 23.5% |
| 11 | CH₂Cl₂ | 24 hours | 25 °C | 20% | 23.5% |
| 12 | Acetonitrile | 24 hours | 25 °C | 17% | 22.4% |
| 13 | CHCl₃ | 24 hours | 25 °C | 21% | 22.7% |
| 14 | THF | 24 hours | 25 °C | 19% | 21.9% |
| 15 | Hexane | 24 hours | 25 °C | 9% | 23.5% |

| | | | | | |
|---|---|---|---|---|---|
| ¹ percentage of grams of crude oil obtained after extraction from the dry plant ² percentage referred to the main cannabinoid content (THC + CBD) in grams per 100 g of extract measured by GC technique. | | | | | |

In view of the results obtained in the optimization of the extraction phase, it was found that extraction with ethanol at room temperature (25°C) for 12 hours was as effective as extraction with heating and that it was the most efficient in terms of cannabinoid extraction performance compared to the use of other solvents for extraction. The establishment and definition of the optimal conditions of the extraction process was followed by a validation phase in which a set of three experiments were performed under such conditions, checking, after analysis of the results, the reproducibility and validity of the process.

The use of methanol as a solvent was also studied, obtaining values of percentage of extraction, temperature and time similar to ethanol. However, this compound was discarded due to its high toxicity.

As a conclusion, the best results were obtained with ethanol as an extraction solvent and during a 12-hour extraction period, as described in the experimental process below.

### Analysis of cannabis samples by NMR

For NMR analysis, a sample of *Cannabis sativa* L. (0.5 g), previously freeze-dried and powdered, is extracted with absolute ethanol (15 mL) at room temperature, with stirring and for 12 hours. After this period, the extract is dried on a chemical drying agent, which is selected from one of the following: anhydrous sodium sulphate or anhydrous magnesium sulphate or anhydrous calcium chloride, filtered through a filter (pore size 0), and concentrated under vacuum drying in a rotary evaporator at 20°C. A sample of this extract (7 mg) is dissolved in deuterated chloroform (3 mL) and analyzed by NMR at room temperature. The analysis is done in duplicate, with a concentration of 15 mg of extract in 3 mL of deuterated chloroform. In all cases the sample is completely dissolved. Under these conditions and for plant samples, the technique has a detection limit of 25 µg/mL, a quantification limit of 75 µg/mL and an absolute error of 4%¹³.

The ¹H-NMR-1H and ¹³C-NMR spectra were acquired in a 400 MHz instrument model ARX400 from Bruker. The residual solvent signal in CDCl3 (δ=7.24 ppm, 400 MHz, for ¹H and δ = 77.0 ppm, 100 MHz, for ¹³C) was used.

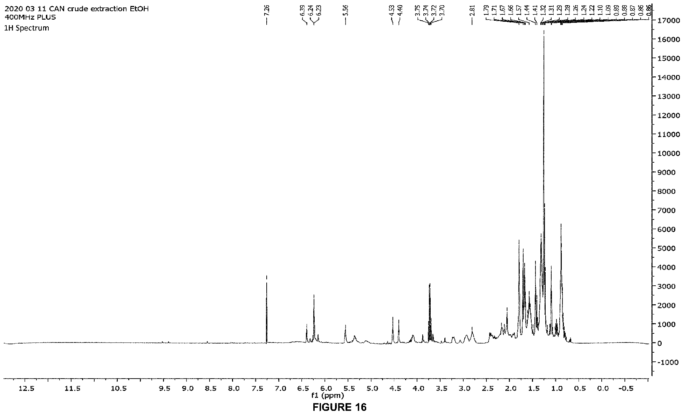

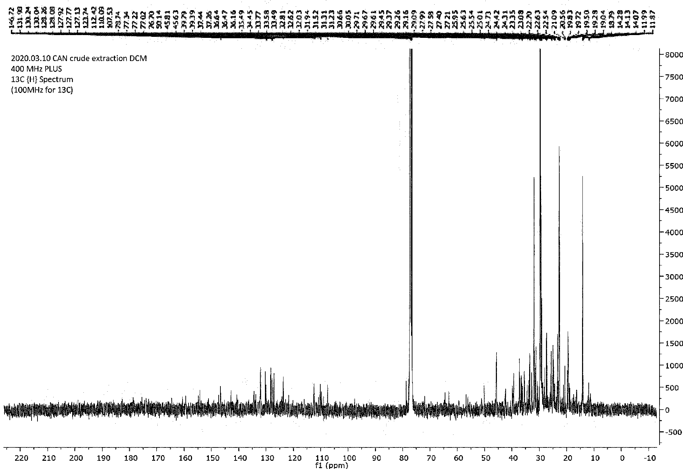

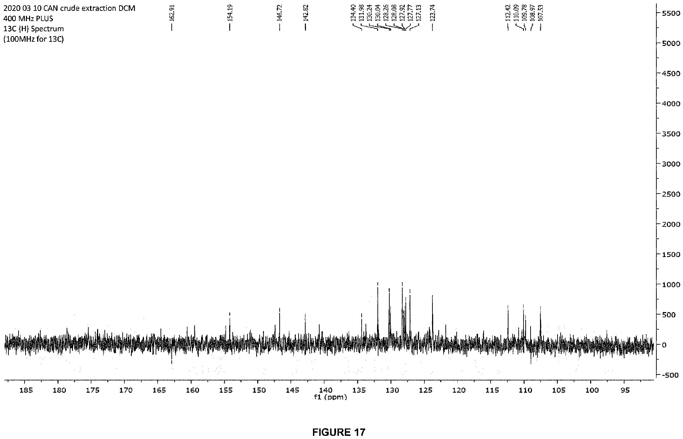

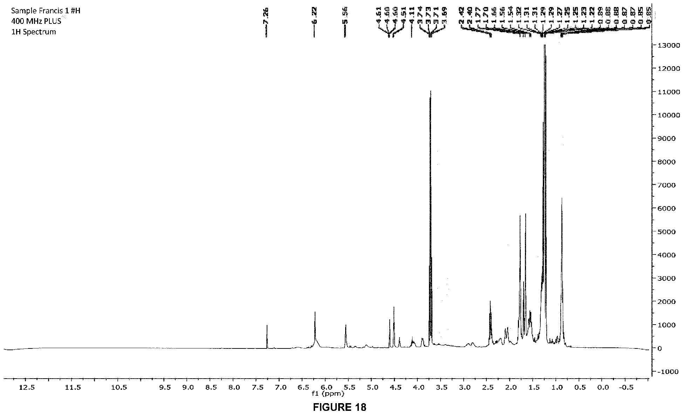

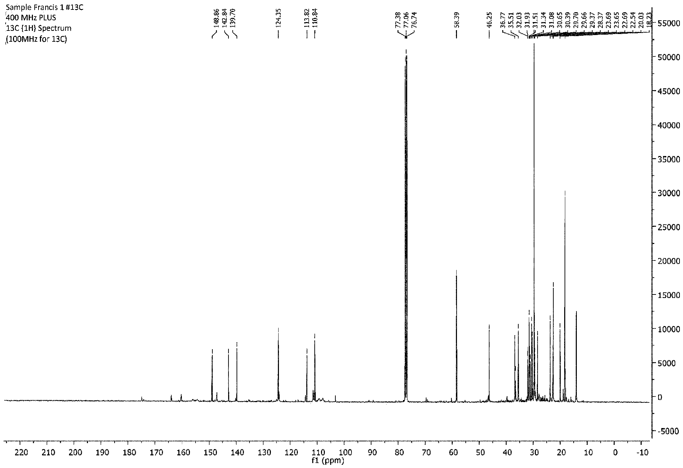

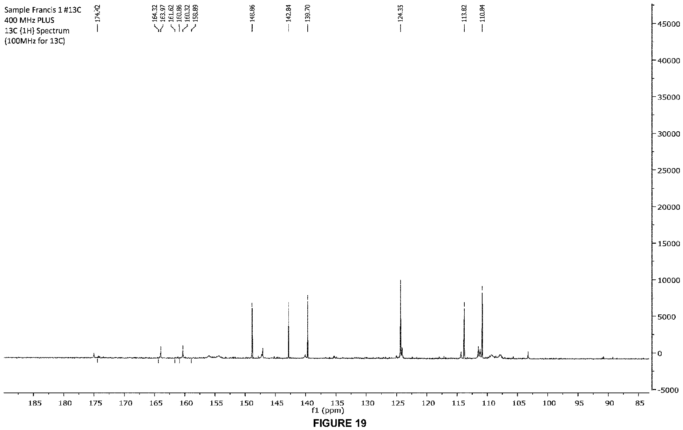

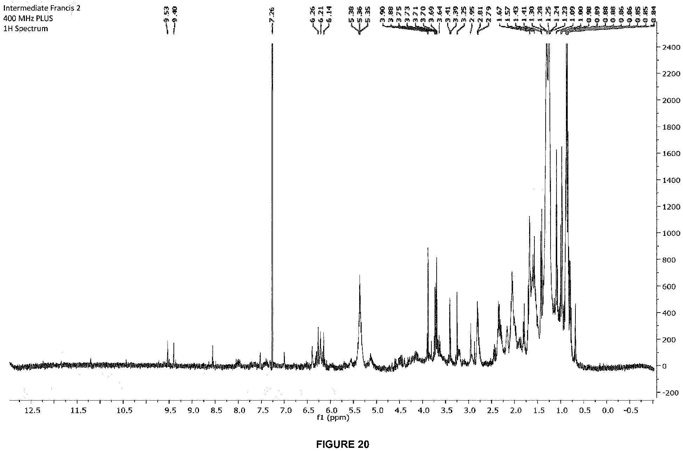

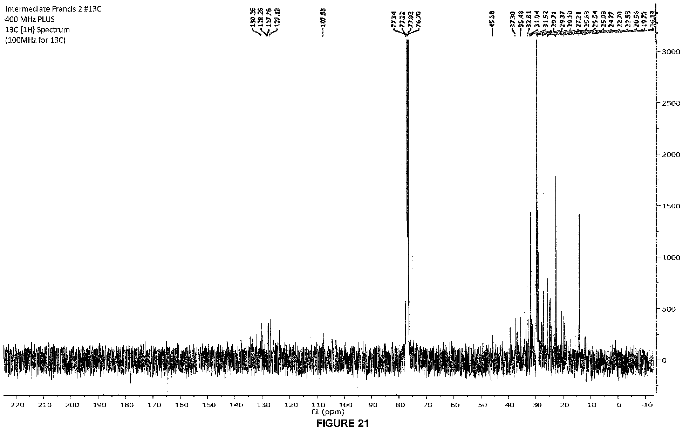

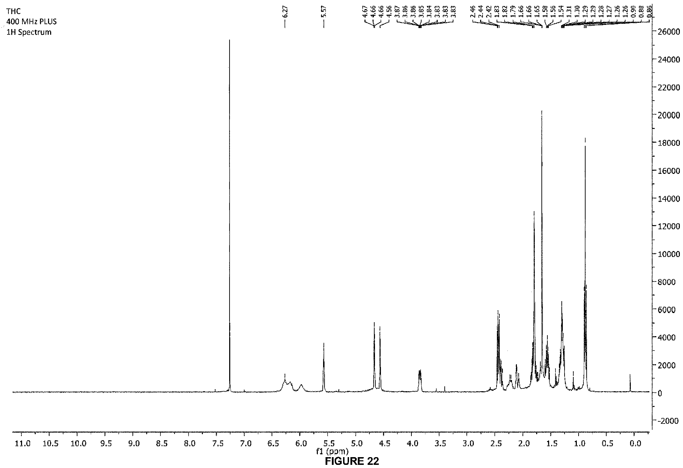

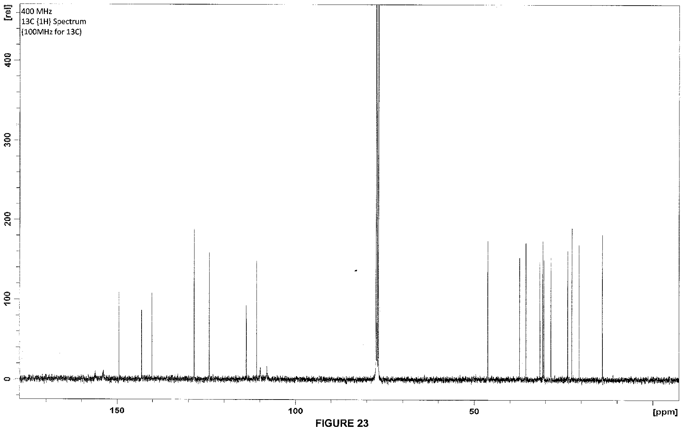

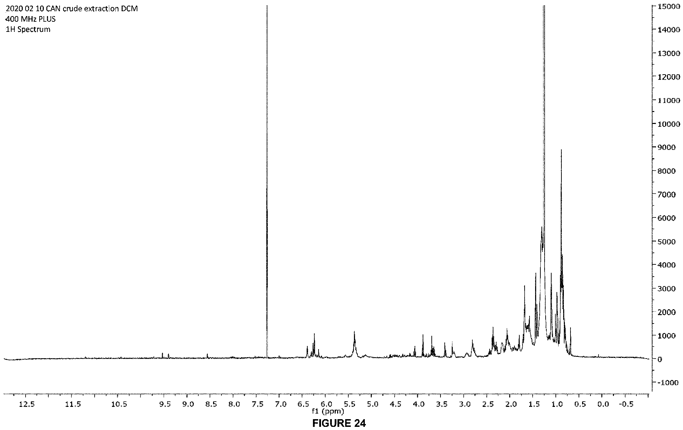

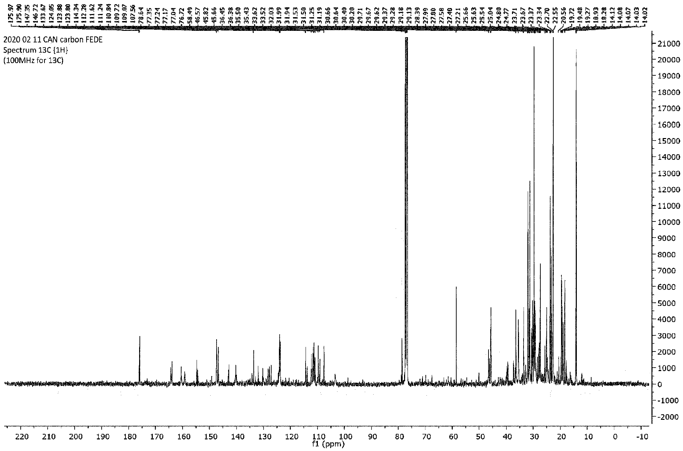

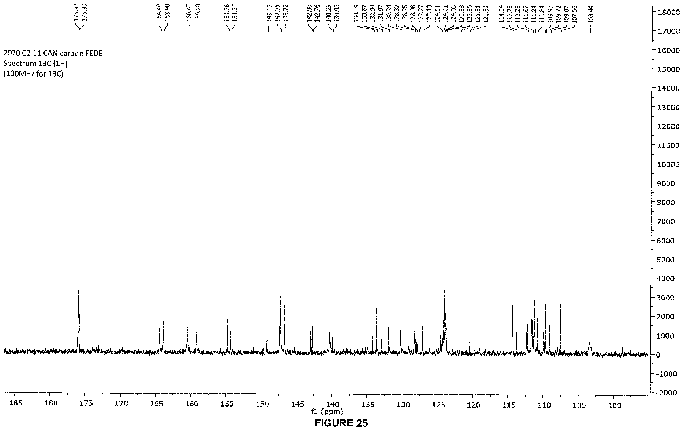

The five analyzed samples present a different pattern in the analyzed area, allowing to assign and correlate the signals found with each sample, finding peaks that are differentiating, which are those highlighted in gray, obtained from figures 16-25. This analysis is shown in TABLE 11.

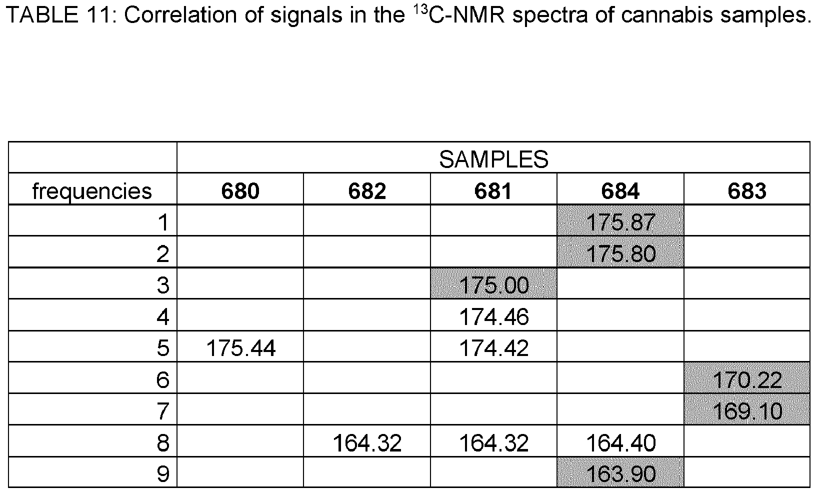

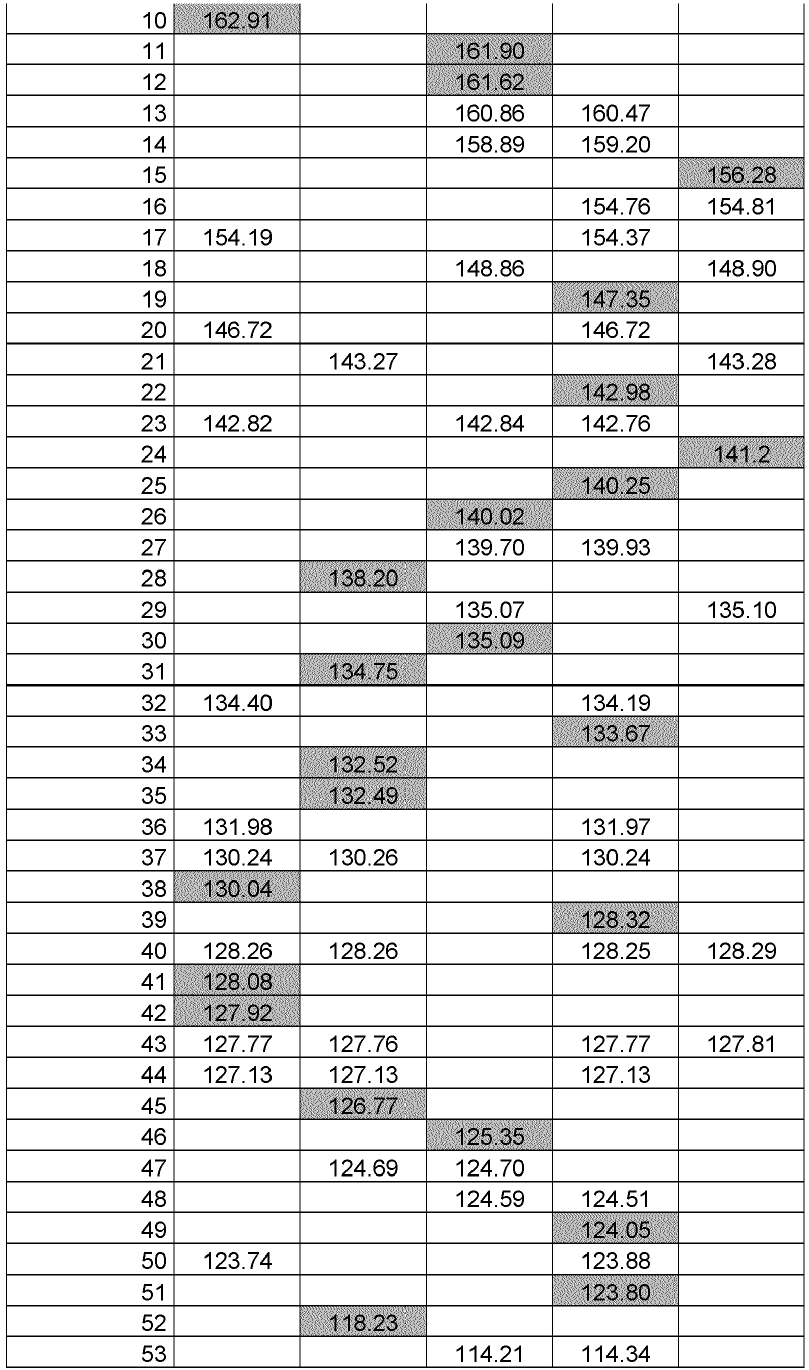

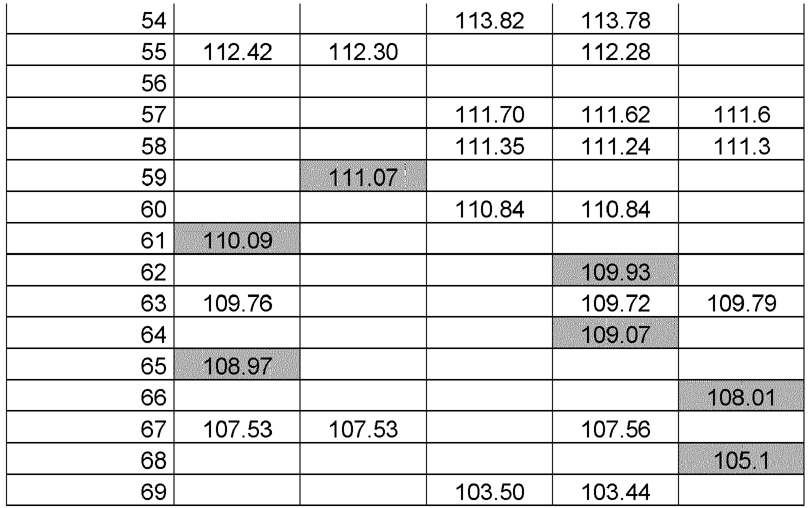

It can be seen that the pattern of chemical composition is very different between the 5 samples analyzed. Table 12 shows the number of signals identified in the ¹³C spectrum of each sample and how many of them were common to the other samples analyzed

**TABLE 12: Matching of identified signals among the cannabis samples tested**

| | | | | | | |
|---|---|---|---|---|---|---|
| | | 680 | 681 | 682 | 683 | 684 |
| | Signals | 20 | 23 | 16 | 14 | 37 |
| 680 | 20 | | 2 | 6 | 3 | 12 |
| 681 | 23 | | | 2 | 4 | 12 |
| 682 | 16 | | | | 3 | 7 |
| 683 | 14 | | | | | 6 |
| 684 | 37 | | | | | |

Thus, obtaining fingerprints by NMR analysis combined with the determination of STR genetic markers in cannabis samples allows to increase the degree of certainty in the identification thereof. Particularly in the case of samples whose genetic pattern is coincident (samples 681 and 684), the chemical composition pattern, obtained based on ¹³C-NMR, is different, confirming the possibility of differentiating samples based on their origin.

The absolute error considered for ppm allocation in each sample is less than 0.01 %. The NMR analysis from the ¹H and ¹³C spectrum of each sample has been performed in triplicate, showing to be reproducible.

### REFERENCES

**1.** Meyer, J. S., & Quenzer, L. F. Psychopharmacology: Drugs, the brain, and behavior 3rd ed. (Oxford, Oxford University Press, 2019).
**2.** National Academies of Sciences, Engineering, and Medicine. (2017). The health effects of cannabis and cannabinoids: The current state of evidence and recommendations for research. National Academies Press.
**3.** WHO Expert Committee on Drug Dependence, Pre-review, "Cannabis plant and cannabis resin: section 1 - Chemistry" (Geneva, 2018).
**4.** Gaviria, C., Zedillo, E., Cardoso, F. H., Romero, A. M., Mockus, A., Garcia, D., ... & Vargas, M. (2009). Drogas y Democracia: hacia un cambio de paradigma. Rio de Janeiro: Comisión Latinoamericana sobre Drogas y Democracia (pág. 7).
**5.** CBOL Working Group. A DNA barcode for land plants. Proc Natl Acad Sci USA 2009; 106(31):12794-7.
**6.** Small E, Cronquist A. A practical and natural taxonomy for Cannabis. Taxon 1976;25(4):405-35.
**7.** Bryant VM, Jones GD (2006) Forensic palynology: current status of a rarely used technique in the United States of America. Forensic Sci Int 163:183-197.
**8.** Brenneisen R, elSohly MA (1988) Chromatographic and spectro- scopic profiles of Cannabis of different origins: part I. J Forensic Sci 33:1385-1404.
**9.** Shibuya EK, Souza Sarkis JE, Neto ON, Moreira MZ, Victoria RL (2006) Sourcing Brazilian marijuana by applying IRMS analysis to seized samples. Forensic Sci Int 160:35-43.
**10.** Shibuya EK, Sarkis JES, Negrini-Neto O, Martinelli LA (2007) Carbon and nitrogen stable isotopes as indicative of geographical origin of marijuana samples seized in the city of São Paulo (Brazil). Forensic Sci Int 167:8-15.
**11.** Howard C, Gilmore S, Robertson J, Peakall R (2008) Developmental validation of a Cannabis sativa STR multiplex system for forensic analysis. J Forensic Sci 53:1061-1067.
**12.** S.Gilmore, R.Peakal, J.Robertson, Short tandem repeat (STR) DNA markers are hypervariable and informative in Cannabis Sativa: implications for forensic investigations, Forensic Sci. Int. 131 (2003) 65-74.
**13.** M. Hsieh, R.J. Hou, L.C. Tsai, C.S. Wei, S.W. Liu, L.H. Huang, Y.C. Kuo, A. Linacre, J.C.I. Lee, A highly polymorphic STR locus in Cannabis Sativa, Forensic Sci. Int. 131 (2003) 53-58.
**14.** D. Balding, Forensic aplications of micro Satelite markers, in D. B. Goldstein and C. Schlotterer (eds.), Microsatelites Evolution and Aplications, (1999) Oxford University Pres, New York, p. 198-210.
**15.** P.S. Keim, K. Zinnamon. DNA fingerprintng for cannabis sativa using STR markers. US2006/0035236A1.
**16.** Xinyi Wang, Peter de B. Harrington, Steven F. BaugH. Comparative Study of NMR Spectral Profiling for the Characterization and Authentication of Cannabis. Journal of AOAC International Vol. 100, no. 5, 2017, 1356*.*
**17.** Wieland Peschel, Matteo Politi. 1H NMR and HPLC/DAD for Cannabis sativa L. chemotype distinction, extract profiling and specification. Talanta 140 (2015) 150-165.
**18.** Miller Coyle H, Shutler G, Abrams S, Hanniman J, Neylon S, Ladd C, Palmbach T, Lee HC (2003) A simple DNA extraction method for marijuana samples used in amplified fragment length polymorphism (AFLP) analysis. J Forensic Sci 48:343-347.
**19.** W. John Kress, Kenneth J. Wurdack, Elizabeth A. Zimmer, Lee A. Weigt, and Daniel H. Janzen. PNAS June 7, 2005 102 (23) 8369-8374.
**20.** Köhnemann, S., Nedele, J., Schwotzer, D., Morzfeld, J., Pfeiffer, H. International Journal of Legal Medicine 126(4), pp. 601-606, 2012.
**21.** Houston, R., Birck, M., Hughes-Stamm, S. et al. Evaluation of a 13-loci STR multiplex system for Cannabis sativa genetic identification. Int J Legal Med 130, 635-647 (2016).

## Claims

1. Method of obtaining a fingerprint of Cannabis samples that comprises the following steps:
a) determination of the genetic profile by the analysis of at least one STR marker,
b) obtaining the chemical profile by means of NMR; and
c) differentiation of cannabis varieties from each other by comparing the results obtained in steps (a) and (b) for each sample with each other and/or with databases of previously obtained fingerprints.

2. Method according to claim 1, wherein the STR marker(s) is/are selected from: D02, C11, H09, B01, E07, 305, 308, B05, H06, 501, CS1, 302, 301, B02, H11 and combinations thereof.

3. Method according to claim 1 or 2, that comprises the following stages:
- extraction and quantification of DNA from the plant sample, and
- amplification of the selected STR by individual PCRs or multiplexed PCRs and analysis of the PCR results.

4. Method according to the preceding claim, wherein the results of:
- individual PCRs are analyzed by agarose gels, and
- multiplexed PCR is analyzed by capillary electrophoresis.

5. Method according to any of the preceding claims, that, comprises the detection of the presence of the Rubisco gene in cannabis samples prior to the determination of the genetic profile by STR marker analysis to confirm that the sample is a plant sample.

6. Method according to claim 1, wherein step b) comprises obtaining the ¹H and ¹³C spectra of the cannabis sample.

7. Method according to the preceding claim, wherein obtaining the NMR chemical profile comprises the following steps: extraction, dissolution in deuterated solvent, obtaining and analyzing the ¹H and ¹³C spectra.

8. Method according to the preceding claim, wherein the sample extraction is performed with a solvent selected from among:
- an alcohol from 1 to 6 carbons,
- one alkane from 1 to 6 carbons and
- an alkane halide derivative,
preferably ethanol and methanol, more preferably ethanol, for a period of time of 8 to 24 hours, preferably 12 hours, and a temperature between 15 and 30°C preferably 25°C.

9. Method according to claim 7, wherein the deuterated solvent is selected from the deuterated variants of: chloroform, acetone, methanol, benzene, heavy water, dimethyl sulfoxide, dichloromethane, trifluoroacetic acid, acetonitrile, pyridine, *N,N-*dimethylformamide and tetrahydrofuran, preferably chloroform..

10. Method according to claim 7, wherein the ¹³C-NMR spectrum analysis comprises at least 40 frequencies (ppm) of peaks associated with metabolites present in the sample, more preferably at least 60 frequencies.

11. Method according to any of the preceding claims, wherein the cannabis sample is selected from *Cannabis sativa* L., *sativa* and *indica* subspecies, *sativa* variety, *Vavilov* spontaneous variety; *indica* (Lam.) variety, *kafiristanica* (Vavilov) variety and combinations thereof, more preferably *Cannabis sativa* L..

12. Method according the preceding claim, wherein the cannabis sample of the plant is selected from leaves, stem, bulb, seeds and flowers, preferably leaves.

13. Method according the preceding claim, wherein the sample amount used to obtain the fingerprint is from 0.1 to 10 g, preferably 0.2 to 1.0 g, more preferably 0.5 g.

14. Database that comprises the fingerprints of cannabis samples obtained by the method described in claims 1 to 13.
